(19) Europäisches Patentamt
European Patent Office
Office européen des brevets

(11) Publication number: **0 453 264 A1**

(12) **EUROPEAN PATENT APPLICATION**

(21) Application number: 91303429.4

(22) Date of filing: 17.04.91

(51) Int. Cl.⁵: **A61M 39/00, F16L 37/12**

(30) Priority: 17.04.90 US 509638
17.04.90 US 509639

(43) Date of publication of application:
**23.10.91 Bulletin 91/43**

(84) Designated Contracting States:
**BE CH DE DK ES FR GB GR IT LI NL SE**

(71) Applicant: Lynn, Lawrence A.
1275 Olentangy River Road Suite 202
Columbus Ohio 43212 (US)

(72) Inventor: Lynn, Lawrence A
1275 Olentangy River Road
Columbus, Ohio 43212 (US)
Inventor: Larkin, Mark E
419 Northgate
Lindenhurst, Illinois 60046 (US)

(74) Representative: Laight, Martin Harvey et al
W.H. Beck, Greener & Co. 7 Stone Buildings
Lincoln's Inn
London WC2A 3SZ (GB)

(54) **Medical connector.**

(57) A medical connector (12) for manually connecting a first fluid conveying conduit (4) in fluid connection with a patient's vasculature and having a junction terminal (8) with a septum (9) at an end thereof to a second fluid conduit (10) for administration of fluid to a patient. The connector is formed of two elements. The first element (22) defines an integral needle hub (24), to which a needle (30) is mounted, a base (26) extending from the hub, and fingers (32,34) extending from the base (26). The second locking element includes a collar (38) with a support and bars (42) extending therefrom so as to be manually slidable along the first element (22) in the direction the needle (30) extends between a retracted and a locking position in which the connector (12) locks onto a junction terminal (8) and the septum (9) penetrated by the needle. The fingers (32,34) flex in a flexure region (62,64) covered by the bars (42,44) in the retracted position. Detents are provided on the base (21) and on a detent carrier (76) formed by slots (80) extending into the collar (38). The bars engage cams (66) on the exterior surface of the fingers (32,34) to flex the fingers. Inner and outer catches (90,92) are provided on the interior surface of each finger (32,34) for respectively engaging larger and smaller septum (9). The catches are configured so that the disengaging force is substantially independent of septum size.

FIG. 4

The invention relates to a medical connector, in particular but not exclusively to a universal medical connector for coupling intravenous conduits.

The attachment of intravenous tubing to intravascular catheters for the administration of fluids and medication to patients has been widely utilized for decades. Generally, an intravenous tubing system comprises a segment of tubing which is distally attached to an intravascular catheter inserted into a patient's blood vessel. Such primary conduits usually have junction terminals at an end which are occluded by a penetrable septum. Such a system acts as a primary conduit system. A secondary conduit may be connected to the primary conduit system for the administration of fluids into the patient. The secondary conduit generally has a fluid source at its proximal end and has an open distal end for attachment to the primary conduit system.

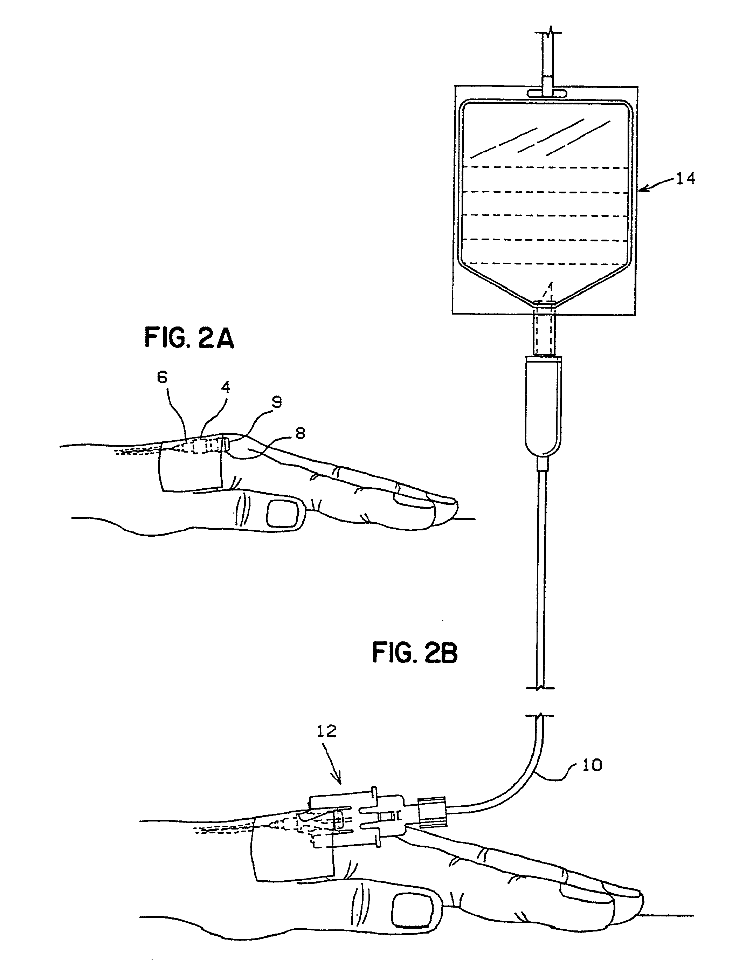

Figure 2a illustrates a conventional primary conduit with its distal end occluded by a septum. In conventional connection, a needle attached to the open end of a secondary conduit is inserted through the septum of the primary conduit to create fluid connection between the fluid source and the catheter.

One major problem with tubing systems is that the needles frequently loosen and become disconnected from the septum during fluid administration. This can result in the medication spilling into the patient's bed or onto the floor. An even greater problem is that the needle may become contaminated through disconnection. Contaminated needle portions may be readvanced through the septum thereby introducing contamination into the primary tubing system.

Yet another problem with existing systems is the danger that the needle poses to health care workers who must daily make numerous connections and disconnections since the needle may be contaminated by viruses which are present in the blood which can often back up into the primary conduit adjacent the septum. Inadvertent needle stick has long represented one of the greatest problems in the medical field. Not only can needle stick occur during connection and disconnection, but needles occasionally become lost in the bed or elsewhere and provide a continual danger to all workers in the hospital environment. The estimated incidence of needle stick injury is 600,000 to 1,000,000 cases per year and may occur particularly when the nurse is using many very different types of intravenous conduit systems.

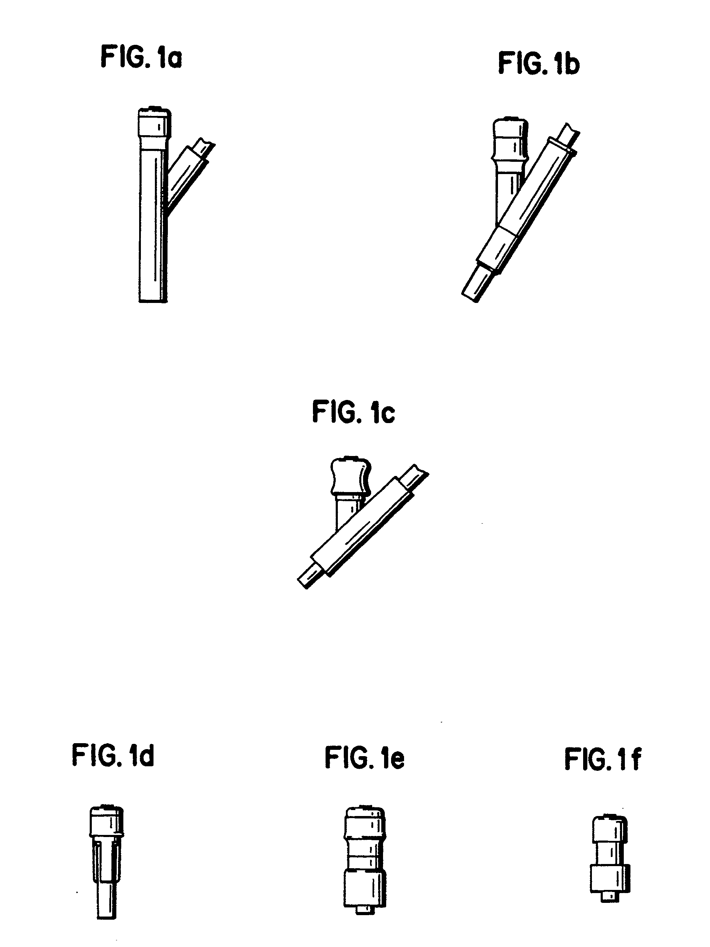

Junction terminals and septae come in a wide variety of shapes and sizes. Figure 1 illustrates the profile of a number of such conventional junction terminals. Commonly, such junction terminals have a head portion which includes a septum which occludes the distal end of the junction terminal. Such junction terminals generally further comprise a rigid tube which extends to the head bearing the septum. The head diameters vary considerably from as little as 0.301 inches to as much as 0.389 inches, and may be comprised of stretched latex or rigid plastic. Similarly the tube diameters vary from 0.224 inches to 0.330 inches. The head diameter may be virtually the same as the tube diameter or may be substantially greater than the tube diameter. This great variation makes very difficult a universal connector which can reliably and effectively connect and tightly hold or lock onto almost everything that is available in the hospital or medical environment, while at the same time providing substantial protection of health workers and patients from needle contact or stick.

The Ogle Patent No. 4,834,716 demonstrates a shielded needle for insertion into a Y-shaped junction terminal. However, the Ogle device provides only a shield and does not provide a secure attachment. Lopez et al., 4,752,292 shows a variety of specifically interfacing devices which include shielded needles intended to provide attachments between primary and secondary tubing systems. However, the disclosed devices have components which must attach to other specific interacting components of a compatible primary system. These devices are not, therefore, compatible with conventional primary systems. Indeed, such devices will not securely attach to the wide range of conventional junction terminals in present use, but rather require the nurse to use a specifically compatible interfacing primary system which may not be readily available. Therefore, none of these devices provides a universal device which can securely lock to the broad range of conventional junction terminals presently in use in medical practice.

According to the present invention in a first, broad, aspect, there is provided a medical connector for manually connecting two fluid conveying systems, comprising: a connector element having a base with a passage or lumen extending through the base, the base having one end adapted to be connected to a secondary fluid conveying system in such a manner that fluid received from the secondary system may flow through the lumen, the connector element further having at least two fingers projecting from the base and defining a space or cavity between the fingers, the lumen terminating within the cavity, and the cavity being adapted to receive into the cavity an end of a primary fluid conveying system to communicate with the lumen so that fluid may flow through the lumen and into the primary system; and a collar manually slidable along the connector element between a retracted position enabling the fingers to lie in a first position in which an end of the primary system may be received into the cavity, and an advanced position flexing the fingers to trap and lock the end of the primary system between the fingers in the cavity.

In accordance with a preferred feature of the invention, it may be arranged that a greater force is required to cause outward flexing of the distal ends of the fingers than is required to cause inward flexing of the distal ends of the fingers.

Preferably each finger is elongate in the direction of projection from the base, but in some circumstances the finger may be a gripping member which is not necessarily elongate in that direction. In preferred forms, each finger has a distal portion or segment connected to the base by a hinge region for providing flexing of the fingers inwardly. Preferably the hinge region is less rigid than the distal portion or segment of the finger.

In accordance with a preferred feature of the invention the collar in the said retracted position thereof has portions overlying at least partially the hinge region, to prevent or inhibit the finger from flexing outwardly. In accordance with another preferred feature, the collar is deformable outwardly to accommodate sliding movement of the collar when engaging distal portions of the fingers. In accordance with another preferred feature, the connector includes a cam on the exterior surface of each finger engageable by the collar in response to sliding movement of the collar towards the said advanced, locking position.

The invention has particular application where the base includes a cannula projecting generally axially from the base relative to a main axis of the connector element, with the said lumen extending through the cannula, the said fingers projecting from the base in a generally axial direction and the said cavity opening in the said axial direction through a distal end of the connector element, the cannula extending from the base towards the said distal end of the connector element and terminating within the cavity, the collar being slidable between the said retracted position, in which position a junction terminal with a septum at an end thereof may be received in the cavity through the opening thereof and the septum may be penetrated by the cannula, and the said advanced position, in which position the fingers trap and lock the junction terminal between the fingers in the cavity.

In accordance with a preferred feature of the invention, the collar includes first and second bars projecting therefrom in a direction towards the distal end of the connector element, and there being provided a cam on each of the fingers engageable with the bars in response to sliding movement of the collar towards the said advanced position to deflect the fingers inwardly towards one another, the bars being flexible outwardly when the fingers are inhibited from flexing inwardly by engagement thereof with an end of a primary fluid conveying system.

In accordance with a further preferred feature of the invention, the fingers are spaced one from the other to define slots there between, and the collar has shield portions overlying at least parts of the said slots in response to movement of the collar into the said advanced locking position to inhibit access to the interior of the connector element.

There will now be set out a large number of alternative aspects of the invention. It is to be appreciated that there will be set out a number of additional broad aspects of the invention independent of those described hereinbefore, and independent of each other. There will also be described a number of preferred and optional features. The scope of the present invention encompasses all combinations of the various novel features set out, whether specifically linked together or whether disclosed separately.

In accordance with another main aspect of the invention there may be provided a universal medical connector for manually connecting a first fluid conveying conduit having a junction terminal with a septum at an end thereof, to a second fluid conveying conduit in fluid connection with a source of fluid for administration to a patient, the second fluid conveying conduit having an open end, comprising: an element including a base and a lumen extending axially through said base, the lumen having a distal end and proximal end, the proximal end of said lumen adapted to couple to the second fluid conveying conduit adjacent the open end of said conduit; the base including a cannula projecting axially from said base, said lumen extending through said cannula for conveying fluid from the second conduit coupled to the proximal end of said lumen through said cannula; said base having at least a pair of oppositely disposed fingers projecting therefrom in a generally axial direction, said fingers defining a cavity therebetween, the cavity opening in said axial direction through a distal end of said element, said cannula extending generally axially from said base toward said distal end of said element, said cannula terminating a tip short of said distal end of said element and within said cavity, said fingers being movable between (i) a first position adapted for receiving therebetween through said opening and into said cavity the junction terminal of the first fluid conveying conduit so that said cannula may penetrate the septum and fluid may flow through said cannula into the first conduit and (ii) a second position to lock the junction terminal between said fingers and said cavity, said fingers being sized and located in said first and second positions thereof so that said cannula is not readily accessible to a human digit, each of said fingers having a distal segment and a connecting segment intermediate said base and said distal segment, said connecting segment defining a hinge region; and a collar manually slidable on and toward said distal end of said element between (i) a retracted position enabling said fingers to lie in said first position in which the junction terminal may be received in said cavity through the opening thereof at the distal end of said element and the septum may be penetrated by said cannula and (ii) a position locking said fingers in said second position thereof to trap and lock the junction terminal between said fingers and in said cavity, said collar engaging said fingers in response to sliding movement of said collar on said element to flex said fingers inwardly along said hinge region toward said second position thereof.

In accordance with a preferred feature a greater force is required to cause outward flexing of the distal segments of said fingers than is required to cause inward flexion movement of said distal segments.

In accordance with another preferred feature said collar in the retracted position thereof, has portions overlying said connecting segments of said fingers and overlying portions of said distal segments of said fingers in said first position thereof so that said distal segments of said fingers are inhibited from flexing outwardly from said first position thereof thereby inhibiting entry of a human digit into said cavity and inhibiting said digit from contacting said cannula.

Preferably the hinge region of said connecting segment of each finger is less rigid than the distal segment thereof. Conveniently, the hinge region of said connecting segment of each finger is positioned along the element proximal to the tip of said cannula.

In accordance with a preferred feature the hinge region extends axially along said finger a predetermined distance and is of reduced width or thickness or durometer in comparison with the width or thickness or durometer, respectively, of said distal segment so that flexion of said hinge region can occur along a variably curved path. In accordance with another preferred feature the hinge region is resilient such that the distal segment rebound to near said first position after said collar has been retracted from the advanced position. In accordance with another preferred feature said collar is deformable outwardly to accommodate sliding movement of said collar portions when engaging said distal segment portions of said fingers.

In accordance with another preferred feature there may be included a cam on the exterior surface of the distal segment of each finger and engageable by said collar in response to sliding movement of said collar toward said locking position, said engagement operating to flex said fingers inwardly toward one another and into said second position to lock the junction terminal between said fingers.

Preferably said collar portions comprise first and second bars projecting from said collar, said bars being deformable to flex outwardly, when said fingers are inhibited from flexing inwardly by engagement thereof with the junction terminal in said cavity, in response to sliding movement of said collar along said element toward said locking position, said engagement operating in accordance with the diameter of the junction terminal received into said cavity.

In accordance with a preferred featured there may be provided a cam on the exterior surface of the distal segment of each finger and engageable by said collar in response to sliding movement of said collar toward said locking position to flex said fingers inwardly toward one another in accordance with the size of the junction terminal received in said cavity and into said second position to lock the junction terminal between said fingers, said collar portions comprising first and second bars projecting from said collar, said bars being deformable to flex outwardly, when said fingers are inhibited from flexing inwardly by engagement thereof with the junction terminal in said cavity, in response to sliding movement of said collar along said element toward said locking position and movement of said bars along said cams.

Preferred forms may include any one or combination of the following features, namely that: said cam comprises a ramp sloping outwardly in the direction of the distal end of said element: that said ramp has a width less than 4 millimetres; said ramp has a width greater than 0.5 millimetres; that the height of said ramp is less than 5 millimetres; that said cam terminates short of the distal end of said finger or that each cam is spaced at least 1 millimetre from the distal end of said fingers and less than 10 millimetres therefrom.

In accordance with a preferred feature said base is provided with at least one detent portion and said collar is provided with a corresponding detent portion for engaging said detent portion on said base to maintain said element sand said collar in one of said retracted and locking positions. Conveniently the base is provided with at least one stop which engages said collar for preventing further sliding of said collar when said collar reaches said locking position. Also conveniently said base is provided with a second stop separated from said first stop, said second stop being engageable with said collar for preventing further sliding movement of said collar when said collar reaches said retracted position.

In accordance with another preferred feature said collar includes a support portion and said collar further includes portions comprising first and second bars extending from one end of said support portion to in part overlie said fingers, said support portion having a shield portion extending from said one end of said support portion between said bars to protect said needle from contamination. Preferably said first and second bars are deformable to flex outwardly, when said fingers are inhibited from flexing inwardly be engagement thereof with the junction terminal in said cavity, in response to sliding movement of said collar along said element toward said locking position, said support portion extending toward said distal end of said element, said shield portion being spaced from said bars by a slot on each side of said shield portion thereby affording flexibility to said bars. Conveniently said bars and the distal segments of said fingers are arcuate with each distal segment and the overlying bar being concentric. Also conveniently said fingers have a plurality of hinge regions separated from each other along the length of said fingers so that large junction terminals are grasped by flexing of said fingers inwardly at a plurality of angles.

Conveniently said cannula comprises a needle. Conveniently said connecting segment has a length greater than 1 millimetre, and said connecting segment has a length less than 10 millimetres, preferably less than 5 millimetres, and most preferably less than 3 millimetres.

Preferably said collar and said element each have a maximum length dimension greater than its maximum width dimension in respective directions normal to said axial direction.

In accordance with another preferred feature said collar has portions overlying said connecting segments of said fingers and overlying portions of said distal segments of said fingers in said first portion thereof, said collar portions and said fingers being arcuate and interrupted by flat sides of said collar and said element, respectively, whereby said connector has opposed flat sides affording a low profile and enabling it to lie flat against a surface.

In accordance with another preferred feature said base has a detent along an exterior surface thereof, said collar having a detent carrier formed by slots extending inwardly from the end of said collar remote from the distal end of said element and on opposite sides of said carrier, and a detent on said carrier for engaging the detents on said base.

In accordance with another preferred feature said distal segments of said fingers have inner and outer axially spaced catches along the interior surfaces thereof for trapping and locking respective junction terminals of different sizes.

In accordance with another preferred feature said base has a catch provided on the exterior surface thereof, said collar having a lever pivotally attached thereto and having a catch engageable with said catch on said base, said lever being pivotable by applied manual force so that said collar can slide along said base.

In accordance with another preferred feature said base has a detent and said collar has a corresponding detent for engaging said base detent, said detents configured such that the force required to move the collar from the retracted position to the locking position is less than the force required to move the collar from the locking position to the retracted position.

In accordance with another preferred feature the force required to move the collar gradually increases as said collar is advanced toward said locking position, said force gradually increasing as said collar is advanced along a distance of greater than 0.5 millimetres.

In accordance with another main aspect of the invention there may be provided a universal medical connector for manually connecting a first fluid conveying conduit having a junction terminal with a septum at an end thereof, to a second fluid conveying conduit in fluid connection with a source of fluid for administration to a patient, the second fluid conveying conduit having an open end, comprising: an element including a base and a needle hub rigidly connected thereto, said needle hub having one end adapted to couple to the second fluid conveying conduit adjacent the open end of second conduit, a needle carried by said needle hub having one end adapted to couple to the second fluid conveying conduit adjacent the open end of the second conduit whereby fluid received from the second fluid conveying coupled to the one end to said needle hub may flow through said needle; said having fingers projecting therefrom in a generally axial direction, said fingers defining a cavity therebetween opening in said axial direction through the distal ends of said fingers defining the distal end of said element, said needle extending generally axially from said hub toward said distal end of said element and between said fingers, said needle terminating in a tip short of said distal end of said element and the distal ends of said fingers, said fingers being movable between (i) a first position adapted for receiving therebetween through said opening and into said cavity the junction terminal of the first fluid conveying conduit so that said needle may penetrate the septum and fluid may flow through said needle into the first conduit and (ii) a second position to lock the junction terminal between said fingers and in said cavity, said fingers being sized and located in said first and second positions thereof so that said needle is not readily accessible to a human digit, each of said fingers having a distal segment and a connecting segment intermediate said base and said distal segment and defining a hinge region; and a collar manually slidable on and toward said distal end of said element between (i) a retracted position enabling said fingers to lie in said first position in which the junction terminal may be received in said cavity through the opening thereof at the distal end of said element and the septum may be penetrated by said needle and (ii) a position locking said fingers in said second position to trap and lock the junction terminal between said fingers and in said cavity, said collar engaging said fingers in response to sliding movement of said collar on said element to flex said fingers inwardly along said hinge region toward said second position thereof; said hinge region of said connecting segment of each finger being less rigid than the distal segment thereof; said collar including first and second bars projecting therefrom in a direction toward the distal end of said element; a cam on each of said fingers and engageable by said bars in response to sliding movement of said collar toward said locking position to flex said fingers inwardly toward one another in accordance with the size of the junction terminal received in said cavity and into said second position to lock the junction terminal between said fingers; said bars being flexible outwardly, when said fingers are inhibited from flexing inwardly by engagement thereof with the junction terminal in said cavity, in response to further sliding movement of said collar along said element toward said locking position.

Preferably on each finger, said cam comprises a ramp sloping outwardly along the exterior surface of said distal segment in the direct of the distal end of said element. Also preferably said connecting segments of said fingers are said bars engaging the distal segments of said fingers are formed respectively, such that a greater force is required to cause outward flexing of the distal segments of said fingers than is required to cause movement of said fingers from said first position toward said second position. Preferably the hinge region of said connecting segment of each finger is less rigid than the distal segment thereof.

In accordance with a preferred feature, said base is provided on its exterior surface with at least one detent portion and said collar is provided on its interior surface with a corresponding detent portion for engaging said detent portion on said base to maintain said element and said collar in one of said retracted and locking positions. Preferably the base is provided on its outer surface with at least one stop which engages said collar for preventing further sliding of said collar when said collar reaches said locking position. Also preferably said base is provided on its outer surface with a second stop separated from said first stop, said second stop being engageable with said collar for preventing further sliding movement of said collar when said collar reaches said retracted position.

Preferably said collar includes a support portion, said first and second bars extending from one end of said support portion, said support portion having a shield portion extending from said one end of said support portion between said bars to protect said needle from contamination. Preferably said bars are deformable to flex outwardly, when said fingers are inhibited from flexing inwardly by engagement thereof with the junction terminal in said cavity, in response to sliding movement of said collar along said element toward said locking position, said collar including a support portion and a shield portion extending from said support portion toward said distal end of said element, said shield portion being spaced from said bars by a slot on each side of said shield portion thereby affording flexibility to said bars. Conveniently, said bars and the distal segments of said fingers are arcuate with each distal segment portion and the overlying bar being concentric. In a preferred form said cannula comprises a single needle, and said base includes a hub disposed about said proximal end of said lumen, said hub being provided with a flange adapted for connection to the open end of the second conduit.

In accordance with another main aspect of the invention there may be provided a universal medical connector for manually connecting a first fluid conveying conduit having a junction terminal with a septum at one end thereof, to a second fluid conveying conduit in fluid connection with a source of fluid for administration to a patient, the second fluid conveying conduit having an open end, comprising: an element including a base and a needle hub rigidly connected thereto, said needle hub having one end adapted to couple to the second fluid conveying conduit adjacent the open end of second conduit, a needle carried by said needle hub for conveying fluid therethrough whereby fluid received from the second fluid conveying conduit coupled to the one end of said needle hub may flow through said needle; said base having fingers projecting therefrom in a generally axial direction, said fingers defining a cavity therebetween, opening in said axial direction between the distal ends of said fingers at the distal end of said element, said needle extending generally axially from said hub toward said distal end of said element and between said fingers, said needle terminating in a tip short of said distal end of said element and the distal end of said fingers, said fingers being movable between (i) a first position adapted for receiving therebetween through said opening and into said cavity the junction terminal of the first fluid conveying conduit so that said needle may penetrate the septum and fluid may flow through said needle into the first conduit and (ii) a second position to lock the junction terminal between said fingers and said cavity, said fingers being sized and located in said first and second positions thereof so that said needle is not readily accessible to a human digit; and a collar manually moveable on said element between (i) a retracted position enabling said fingers to lie in said first position in which the junction terminal may be received in said cavity through the opening thereof at the distal end of said element and the septum may be penetrated by said cannula and (ii) a position locking said fingers in said second position thereof to trap and lock the junction terminal between said cavity, said collar engaging said fingers in response to movement of said collar on said element to move said fingers inwardly along said second position thereof; said fingers being spaced one from the other to define slots therebetween, said collar having shield portions overlying at least portions of said slots in response to movement of said collar into said locking position to inhibit contact with potentially contaminated structures through the slots.

Preferably said collar includes first and second bars projecting therefrom; a cam on each of said fingers and engageable by said bars in response to movement of said collar toward said locking position to flex said fingers inwardly toward one another in accordance with the size of the junction terminal received in said cavity and into said second position to lock the junction terminal between said fingers; said bars being flexible outwardly, when said fingers are inhibited from flexing inwardly by engagement thereof with the junction terminal in said cavity, in response to further movement of said collar along said element toward said locking position.

Also preferably, said collar and said element each have a maximum length dimension greater than its maximum width dimension in respective directions normal to said axial direction, said bars and said fingers are arcuate and concentric and are interrupted by flat sides of said collar and said element, respectively, whereby said connector has opposed flat sides affording a low profile and enable it to lie flat against a surface.

In accordance with another main aspect of the invention there may be provided a universal medical connector for manually connecting a secondary fluid conveying tubing system to a junction terminal of a primary fluid conveying tubing system, the primary tubing system being in a fluid connection with a patient's blood vessel or body cavity, the universal medical connector comprising: an element having a proximal end and distal end and including a base with a lumen extending axially through the base, the base having one end adapted to connect to the secondary tubing system whereby fluid received from the secondary tubing system coupled to the one end of the base may flow through the lumen, said element further having a plurality of disposed fingers projecting from said base, the fingers being connected to said base and extending in a generally axial direction to define distal rigid finger segments, the fingers further defining a central cavity intermediate said fingers, the cavity opening in an axial direction through said distal end of the element, said lumen terminating within said cavity, the fingers being movable by flexion into said cavity between (i) a first position adapted for receiving through said opening and into said cavity the junction terminal of the primary tubing system to engage said lumen so that fluid may flow through said lumen into the primary tubing system; and (ii) a second flex position to lock the junction terminal between said rigid finger segments and in said cavity; said element having a hinge region proximal to said rigid finger segments, said elements being more easily deformed along said hinge region than along said distal finger segments; and a collar having a proximal and distal end manually slidable about said element and toward said distal end of said element between (i) a retracted position enabling said fingers to lie in said first position in which the junction terminal may be received in said cavity through said opening to engage said lumen so that fluid may flow from said lumen into said junction terminal, and (ii) an advanced position flexing said fingers in said hinge to trap and lock the junction terminal between said fingers and in said cavity, said collar engaging said fingers in response to sliding movement of said collar on said element to flex said fingers inwardly.

Preferably the junction terminal terminates in a blind end, the hinge region positioned along said element so that at least a portion of said hinge region is distal (relative to the element) to said blind end when the junction terminal is maximally advanced into said cavity. Also preferably the hinge region is positioned along said element so that at least a portion of said hinge region is distal (relative to element) to said element when said junction terminal is maximally advanced into said cavity.

The present invention relates, at least in preferred forms, to an improved connector which is simple, economical to manufacture and which reliably connects together virtually any junction terminal having a septum to any conduit having an open end. The device can even interface with a junction terminal which is completely covered except for its tip by tape against the skin as shown in Figure 2(b).

Yet further preferred and optional features and combinations of features will now be described. It is particularly to be appreciated that the following statements relate only to preferred or optional or exemplary features and do not set out essential features of the invention.

The connector is conveniently formed of two elements slidable with respect to each other to lock and disengage two conduits. In the universal connector of the present invention, a flange on a needle hub may be adapted to couple to the open end of a fluid conveying conduit which typically comes from an elevated bag or bottle or from a pump. The hub is in fluid connection via a bore to a single needle mounted to the other end of the needle hub. Extending from the needle hub is a base which defines a passage through which the needle extends. The base is provided with a plurality of fingers which extend therefrom along the direction the needle extends toward an open end remote from the hub.

The fingers bound a space or cavity into which the end of a junction terminal can be received through the open end of the space such that the needle penetrates the septum. The fingers further have a hinge or flexing line or joint or flexure region between the base and fingertip in which flexing can occur. The region may be a weakened segment between the open end and the base. This weakened segment can comprise a segment of reduced width and/or thickness or can be provided by prestressing a portion of the fingers during manufacture.

The weakened segment is in effect a connecting segment proximal to the distal segments of each finger, with the connecting segment being less rigid than the distal segments. The connecting segment may be as little as 0.5mm in length so as to provide a single hinge line or joint, or as long as 10mm in length to provide a smooth and variably arcuate flexure for accommodation of variable head and tube diameters.

A locking element in the form of a collar is manually slidable from a retracted position in which the septum may be inserted into the space and penetrated by the needle to a locking position flexing the fingers at the connecting segment to trap and lock the septum in the space. The collar is elastically and forceably deformable to accommodate sliding along the fingers toward the locking position. When the collar is slid back to its retracted position, it returns substantially to its original shape. The length of the collar in the direction the needle extends is preferably greater than its transverse diameter.

The connecting segment is covered by the collar in the retracted position so that the fingertips are prevented from flexing outward to any substantial extent so that a human digit is inhibited from entering into the space and potentially sustaining contact with or injury by the needle. Preferably, the fingers are configured so that a human digit cannot readily enter the space to contact the needle in either the retracted or locking position.

Thus, the connector by a simple one-handed motion can be locked onto and released from a junction terminal. Moreover, the connector can lock onto virtually any of the junction terminal configurations which are in use. At the same time, substantial protection against needle contact or stick injury is provided. It is the elastic deformability of the collar which accommodates to the sliding of the collar along the base into the locking position over heads of large diameter whereas the rigidity of the distal segments of the fingers resist outward flexing and deformation to inhibit inadvertent insertion of a human digit.

According to one aspect of the invention, the collar is formed with a plurality of bars extending toward the open end with the bars being relatively rigid but forcefully deformable to flex outwardly as they slide along the fingers. Preferably, the fingers are each provided with a cam portion on the exterior surface thereof to respectively engage the interior surface of a bar so that the fingers can flex inwardly in accordance with the size of the junction terminal trapped and locked by the fingers. The bars can flex outwardly as they engage the cam portion when the fingers are inhibited from flexing inwardly by a junction terminal of large diameter since the wedge effect of the cam can force the rigid bars into external flexion.

Using cantilevered bars rather than a solid ring provides several important advantages. First, the cantilevered construction allows the connector to lie flat against a patient's skin. The profile of the connector is thus relatively low. Alternatively, when the junction terminal is covered by tape, the connector can be orientated so that the tape can simply slide into the space and extend out of the connector between the bars. Also, the bars provide more spring-like deformation than does a solid ring.

According to a further aspect, the cam portion provided on the exterior surface of each finger which engages a bar as the locking element is slid into the locking position, preferably, is formed as a narrow ramp sloping outwardly in the direction of the open end and having a narrow width. The slope of the ramp is preferably about 30 degrees. The width is preferably less than 3mm. Providing a narrow ramp in this fashion reduces the frictional engagement forces between the interior surface of the bars and the cam, and thereby permits the collar to be slid to the locking position with relatively little force. This reduces the transmission of force to the junction terminal within the space and thereby may reduce the transmitted force to the catheter connected to the terminal. In many cases, the junction terminal, the catheter, and the blood vessel are in close approximation (as shown in Fig. 2(b)) so that minimum transmission of force to the junction terminal is critical to prevent the transmission of force to the vein through the catheter, which force could damage the vein.

The cam is preferably spaced at least 1mm from the fingertip and less than 10mm from the fingertip end so that a greater flexing arc of the tips of the fingers is achieved with less height of the cam, thereby further reducing the profile of the connector.

Preferably, the base is provided with a first stop on its exterior surface for preventing further sliding of the collar once it has reached the locking position and a second stop separated from the first for preventing further sliding of the collar once it has reached the retracted position. Placing the stops on the base ensures that they are not affected by the flexing movement of the bars or of the fingers.

Further, a detent is provided on the exterior surface of the base with a corresponding detent being provided on the interior surface of the collar.

According to a further aspect of the present invention, the collar is provided with a detent on a detent carrier which engages a corresponding detent and stop on the outer surface of the base. The carrier is preferably formed by slots extending inwardly from the end of the collar. The detent carrier acts like a spring, flexing as it is pushed past the corresponding detent. Thus, the collar can be pushed back and forth innumerable times between retracted and locking positions without wearing out the detent or stops.

According to a further aspect of the present invention, each finger is provided with separated catches on its interior surface for engaging the junction terminal and septum. The inner catch primarily engages larger junction terminals and the outer catch engages the conduit for both larger and smaller junction terminals. The catches are configured so that inadvertent disengaging force to separate the two elements is less dependent on junction terminal diameter. The head of the junction terminal therefore releases at a given range of pull forces upon the coupler which is largely independent of junction terminal diameter. This release mechanism prevents an inadvertent forceful pull on the secondary intravenous tube connected to the coupler (such as might occur when the secondary intravenous tube becomes wrapped around a bed rail) from damaging the junction terminal.

The release mechanism is provided by the relationship between the pair of catches provided along the interior surface of the distal end of the fingers, and the cam interaction with the bars. More particularly, the inner catch, which engages the large junction terminal, gradually slopes in the direction of the open end. Since a junction terminal of larger head diameter will be held by a stronger spring force of the bars, the catch angle is less acute to reduce the holding force against inadvertent displacement. Smaller head diameters result in less spring force and, therefore, a sharper angle is provided with the outer catch to increase the holding force against such displacement.

The sharper angle of the outer catch does not substantially hold the larger diameter terminal because this catch is held away from the head by the more proximal inner catch which abuts the side of the head during displacement. Thus, the connector releases at roughly the same applied force which is less dependent of junction terminal diameter.

According to yet another aspect of the present invention, a shield portion is provided on the collar extending distally in the direction of the open end and situated between the bars to protect against droplet nuclei contamination of the needle such as might occur if a hospital worker would cough or sneeze in the vicinity of the connector before engagement with the junction terminal.

The fingers may be provided each with a single weakened segment or alternatively with a plurality of weakened segments separated from each other along the length of the fingers. With a plurality of weakened serpents, a large septum can be grasped by the flexing of different segments at different angles and at multiple points along the fingers.

Embodiments of the invention will now be described by way of example with reference to the accompanying drawings in which:-

Figure 1 shows the profile for a number of typical septae and terminals illustrating the variable configurations.

Figure 2(a) shows a conventional primary conduit, in this case a short heparin well, connected to a catheter within a patient's vein. The primary conduit is shown covered by tape except for the tip bearing the septum.

Figure 2(b) shows the primary conduit of Figure 2(a) connected to a secondary conduit by the connector of the present invention.

Figure 3 shows a side view of the assembled combination.

Figure 4 shows a top view with the locking element separated from the element defining the needle hub, base, and fingers.

Figure 5 shows a top view of the assembled combination with the locking collar in the retracted position.

Figure 6 shows a sectional view of Figure 5 along the lines 6-6.

Figure 7 shows a top view of the assembled connector in the locking position.

Figure 8 shows an end view of Figure 5.

Figure 9 shows a sectional view of the assembled connector coupling an open ended conduit to a junction terminal and septum having a large head and a large tube.

Figure 10 shows a sectional view of the assembled combination coupled to a junction terminal having a smaller head and tube, and wherein the head diameter is minimally greater than that of the tube.

Figure 11 shows a sectional view of the assembled combination with the locking element in the locking position upon a Y-shaped junction terminal.

Figure 12 shows a sectional view of the assembled combination and Y-shaped junction terminal of Figure 9 with the locking element being retracted.

Figure 13 shows the assembled combination and Y-shaped junction terminal of Figures 9 and 10 with the locking element fully retracted against the proximal stop.

Figure 14 shows a detailed sectional view illustrating the proximal stop and the detents.

Figure 15 illustrates a detailed view of the inner and outer catches on each finger.

Figure 16 shows a further embodiment in which a plurality of flexing regions are provided along the length of each finger.

Figures 17(a) and (b) show a further embodiment in which the detent carrier flexes outwardly when squeezed.

Figure 2(b) shows a view of the unique connector of the present invention forming part of a system for supplying intravenous fluid to a patient. Primary conduit 4 is shown attached to catheter 6 (shown with its tip in the vein of the dorsun of a patient's hand). Primary conduit 4 has a conventional junction terminal 8 having a septum 9 (the junction terminal 8 is shown partially covered by tape as in typical operation.) Secondary conduit 10 is coupled to primary conduit 4 by the universal connector 12 of the present invention, thereby placing bag 14 in fluid communication with the patient's vein.

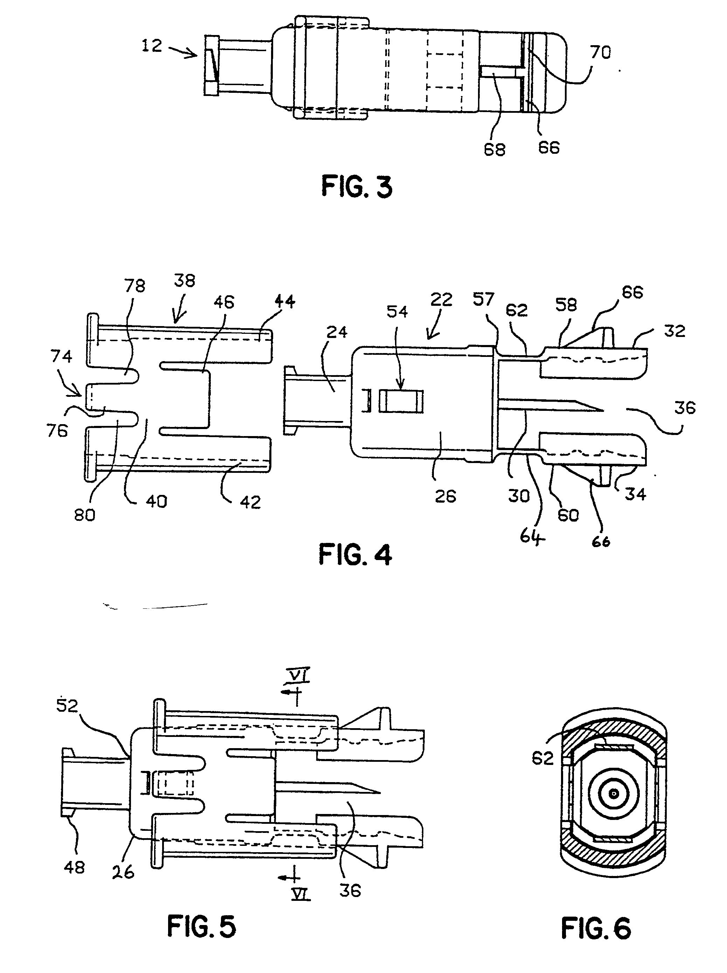

Reference is now made to Figures 3 through 9 which illustrate a preferred embodiment of the present invention. Connector 12 is formed of two distinct elements.

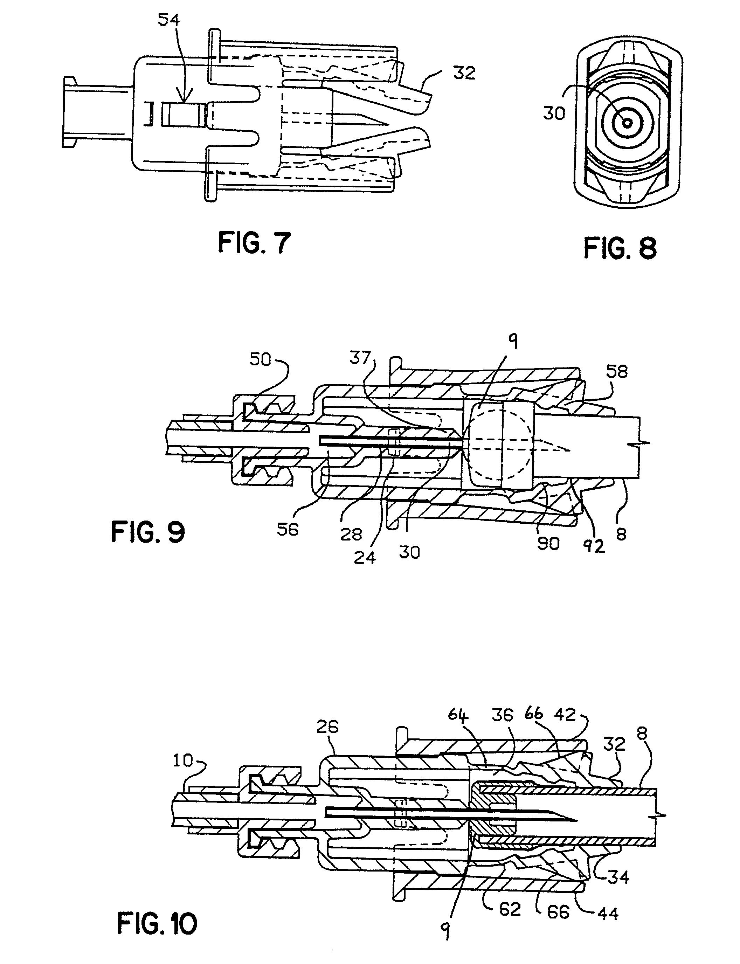

Element 22 integrally forms a needle hub 24, a base 26 having an inner passage 28 as shown in Figure 9 through which a needle 30 extends from the proximal hub 24 and a pair of fingers 32 and 34 extending from the base 26 in the direction that the needle 30 extends and bounding an inner space 36 through which the needle 30 extends. Preferably the base 26 and hub 24 are integral, but may be formed separately and attached by adhesive or otherwise.

The space 36 is preferably cylindrical having a length of 0.661 inches and a diameter of 0.436 inches. The proximal end of space 36 is defined by base 26 comprising septum stop 37 as shown in Figure 9. The length of needle 30 proximal to septum stop 37 is preferably about 0.627 inches. The length of needle 30 from the septum stop to the needle point is about 0.466 inches.

The locking element is integrally formed as a collar 38 having a support portion 40 and further having a pair of arcuate bars 42 and 44 extending therefrom and a pair of shields 46 extending parallel to the bars 42 and 44 from the support portion and separating bars 42 and 44.

Both elements 22 and 38 are formed of a suitable plastic material such as PCTG which is a polyester, a polycarbonate and PCTG blend sold under the trademark EKTAR, or an acrylic multipolymer sold under the trademark CYRO LITE. All these materials are relatively rigid and suitable for use in a medical environment and can be sterilized in accordance with conventional techniques.

Needle hub 24 provides a flange 48 at the proximal end thereof which is readily and conventionally attachable to open ended conduits 50 (Figures 9 and 10) of the type now used in hospitals and elsewhere for connection to bottles and bags of fluid and to pumps providing fluid to be administered into a patient's vein. The distal end 52 of the needle hub 24 remote from the flange 48 is integrally connected to base 26. Base 26 has detent 54 on the exterior surface thereof as can be best seen in Figure 4.

Needle 30 is conventionally coupled to needle hub 24 and extends through a passage 56 in the needle hub 24 as best seen in Figures 9 and 10. A pair of arcuate fingers 32 and 34 integrally extend from the distal end 57 of base 26 (remote from its joinder to needle hub 24) to respective fingertips. Fingers 32 and 34 are formed with distal segments 58 and 60 respectively joined to base 26 by connecting segments defining connecting weakened segments 62 and 64. The connecting segments 62 and 64 are preferably between 1mm and 10mm in length, preferably less than 5mm, and preferably greater than 3mm.

In this embodiment two fingers are provided, but more can be used if desirable or necessary. Preferably the fingers are identical. In operation the fingers flex inwardly with considerably less force than required to make them flex outwardly. Equally important, the fingers spring back to substantially their initial position when the inward force is removed.

Fingers 32 and 34 bound the inner space 36 through which the needle 30 extends and serve to protect users of connector 12 from inadvertent needle stick. On the exterior surface of each of the fingers 32 and 34 is provided a cam portion 66 which is formed as a narrow ramp 68 terminating in a transverse ridge 70 forming with ramp 68 a T-shaped cam portion 66 as can be seen in Figures 3 and 4. Ramp 68 is spaced back from the fingertips of fingers 32 and 34 by a distance which is preferably at least 1mm and no more than 10mm. The width of the ramp is preferably about 0.5mm, but can be 0.4mm or less or, 0.6mm or even greater.

A narrow ramp is advantageous in limiting the force which must be applied to overcome friction when the locking element 22 is manually shifted from the retracted to the locking positions as will be described hereafter. The ramp height is preferably approximately 3mm, but may range from .5 - 5mm or greater depending on the distance the cam is set back from the tips of the fingers. The angle of the ramp is preferably about 30 degrees.

When the collar 38 is assembled with element 22 as illustrated in Figures 5-9, detent 54 engages a corresponding hook-like detent 74 on the interior surface of a detent carrier 76, the carrier 76 is formed by slots 78 and 80 extending into support 40. In operation, detent carrier 76 can flex as collar 38 is slid along base 26.

The arcuate bars 42 and 44 each preferably extend no more than 90° about the periphery of the support and base. Alternatively, the bars may be straight posts. The preferable maximum outward flexing of the bars induced by the wedging force of cam 66 is about 0.0885 inches in this embodiment.

In order to lock the connector of the present invention onto a junction terminal 8 having septum 9, the junction terminal 8 is inserted into the space 36 bounded by fingers 32 and 34 so that the needle 30 penetrates septum 9 of terminal 8 and effects fluid communication between the open end 50 of conduit 10 coupled to needle hub 24 and the catheter 6 coupled to the junction terminal 8 of the primary intravenous conduit 4 as shown in Figure 2(b). When junction terminal 8 is maximally advanced septum 9 will abut septum stop 37 as shown in Figures 9-11.

Figures 9-13 show the connector of the present invention locked onto various kinds of typical junction terminals. For example as shown, when the collar 38 is manually pushed in the direction of the open end of the space 36 through which needle 30 extends, bars 42 and 44 slide along the fingers 32 and 34 and engage cam portion 66 causing fingers 32 and 34 to flex inwardly. A flexure therefore is defined along the connecting weakened segments 62 and 64 intermediate the base 26 and distal segments 58 and 60. For a large junction terminal 8, such as shown in Figure 9, the bars 42 and 44 may, in fact, flex slightly outward as the fingers 32 and 34 flex inward. For a smaller junction terminal 8' as shown in Figure 10, of course, the flexing of the fingers 32 and 34 will be greater inwardly and the flexing of the bars 42 and 44 outwardly less or non-existent. It is the combination of potential outward flexion of the bars and inward flexion of the fingers which allows the connector to lock onto a wider range of junction tube diameters.

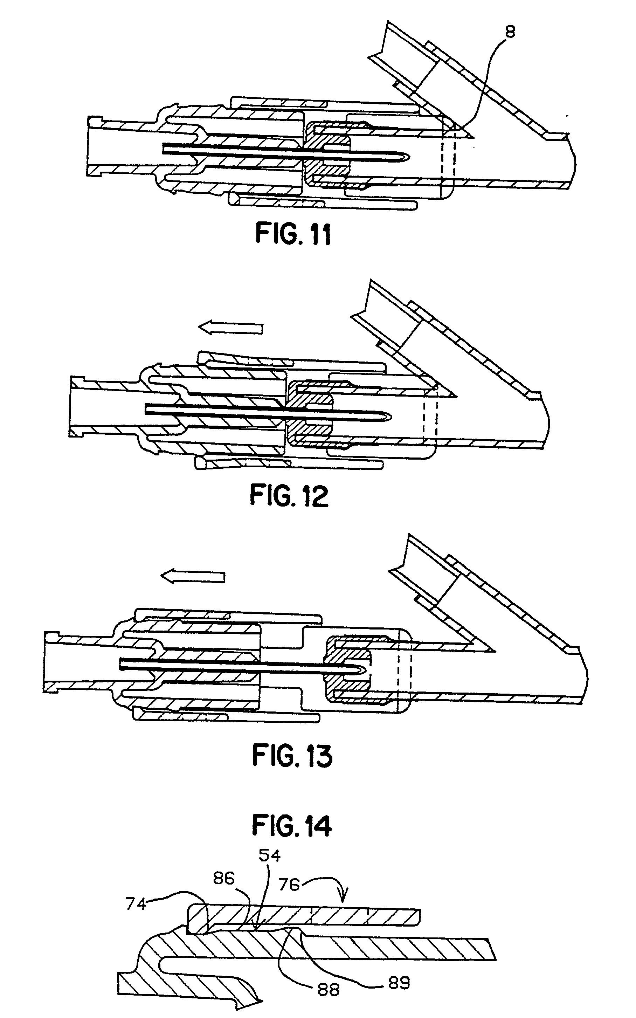

Figures 11 through 13 illustrate how the connector 12 is released as the collar 38 is manually moved between the locking position illustrated in Figure 11 and the retracted position illustrated in Figure 13. The junction terminal 8 in this instance is a conventional Y-junction.

Figure 14 shows in detail the detent 54 on the base 26 and the corresponding detent 74 on detent carrier 76. The detent 74 on the detent carrier 76 comprises a hook-shaped portion extending downwardly as shown from the end of the detent carrier 76. The detent on the base 26 is formed as a proximal portion 86 with a rising slope which gradually rises to a maximum height at a summit 88 and then abruptly declines in a distal portion 89 back to the original elevation. Making the gradual slope in the direction of movement toward the locking position and the abrupt slope in the opposite direction ensures that minimal force only need be applied to slide collar 38 distally thereby reducing the risk of damage to the vein by pushing too hard on the catheter, while at the same time ensuring that much greater force is required to manually slide the collar 38 in the opposite direction to the retracted position so that inadvertent disconnection is avoided.

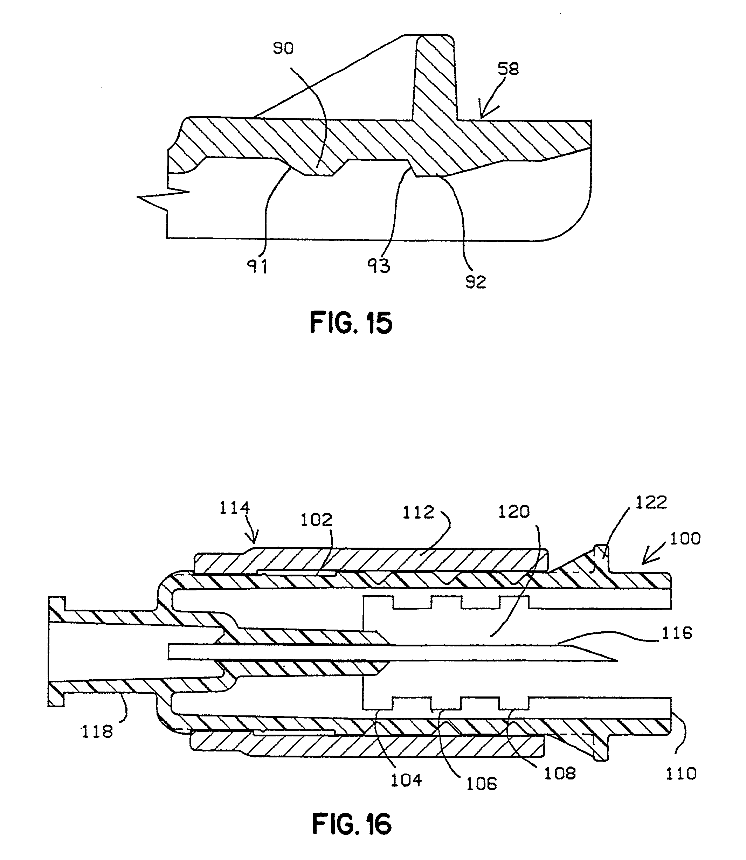

Reference is now made to Figure 15 which illustrates distal segment 58 of one of the fingers 32 and 34 with inner and outer catches 90 and 92. Inner catch 90 is formed with a gradual slope 91 in the direction of the distal end. As shown, for example, in Figure 9, inner catch 90 engages a large junction terminal 8. When a large junction terminal 8 is engaged as shown in Figure 9, the bars 42 and 44 are deformed outward to a considerable extent and the spring force is high. Although the spring force is high, the gradual slope 91 of inner catch 90 compensates so that an inadvertent and extremely forceful pull (such as might occur if the secondary tubing inadvertently became wrapped around a bed rail) will allow the connector 12 to disengage from the junction terminal without destroying the junction terminal. Conversely for a small junction terminal, the spring force is less, but the abrupt slope 93 of catch 92 holds the small junction terminal better. The larger junction terminal 8 is not held by the outer catch 92 during a forceful inadvertent pull because inner catch 90 rides over junction terminal 8 swinging outer catch 92 outwardly. Thus, the force necessary to inadvertently disengage without retracting collar 38 is less dependent of junction terminal diameter because the outer catch 92 has a sharper angle than said inner catch 90.

Figure 16 illustrates an embodiment in which a plurality of weakened regions are provided for gripping a junction terminal by flexing inwardly at different points to provide even greater universality. Each finger 100 extending from base 102 has three weakened regions 104, 106 and 108 between its point of connection to base 102 and its tip 110. As in the other embodiments, regions 104, 106 and 108 are preferably covered by bars 112 extending from collar 114 in the retracted position. Needle 116 extends from hub 118 into space 120 as in the other embodiments. Bars 112 engage cam 122 as described above.

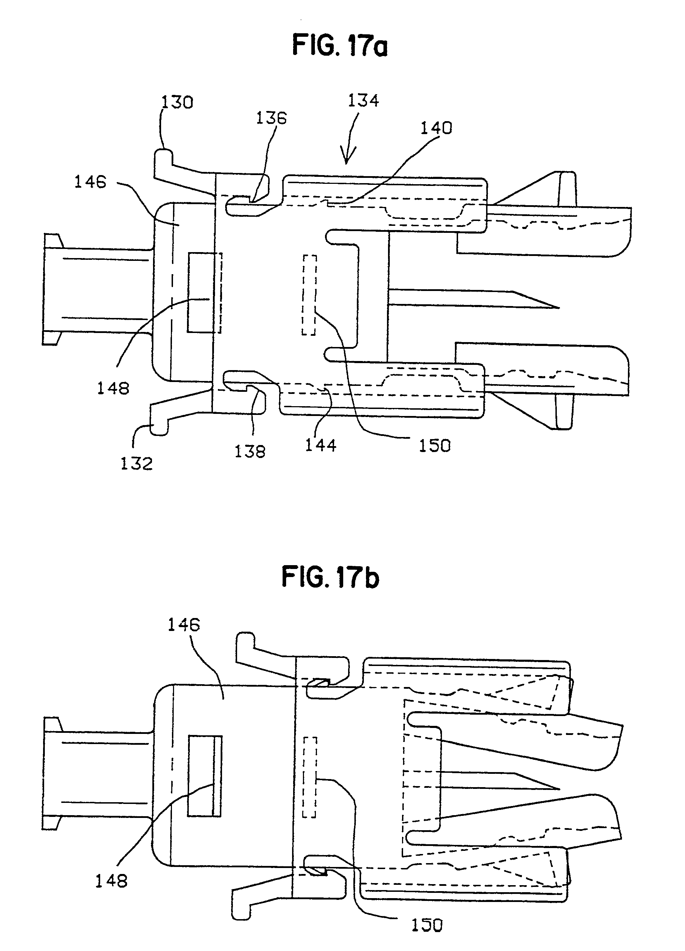

Figures 17(a) and 17(b) illustrate a further embodiment in which the force required to slide the collar to the locking position is further minimized. This is achieved by a pair of attached lever arms 130 and 132 which extend outward from collar 134. Arms 130 and 132 respectively have catches 136 and 138 which are pivoted beyond detent 140 and 144 when arms 130 and 132 are manually squeezed. Alternatively, the collar 134 can be slid with catches 136 and 138 riding over the respective detents without squeezing lever arms 130 and 132. However, collar 134 cannot readily be retracted without squeezing so some further security against inadvertent disconnection is provided. In this extended position, collar 134 can be slid without resistance to the locking position shown in Figure 17(b) engaging detents 142 and 144 of base 146. Stops 148 and 150 are provided on base 146 and function as described above. The other elements and details are as described above.

Many changes and modifications can, of course, be made without departing from the spirit and scope of the invention. For example, the connector could be permanently affixed to an open ended conduit. The connector can be used with junction terminals which have a pre-perforated septum such as a slit for receiving a blunt cannula. Alternatively, the needle may be a blunt cannula which penetrates such pre-perforated septum or slit. Accordingly, the scope of the invention is to be determined by the following claims.

**Claims**

1. A medical connector for manually connecting two fluid conveying systems, comprising:
   a connector element (22) having a base (26) with a passage or lumen (28) extending through the base (26), the base (26) having one end adapted to be connected to a secondary fluid conveying system (10) in such a manner that fluid received from the secondary system (10) may flow through the lumen (28), the connector element (22) further having at least two fingers (32,34) projecting from the base (26) and defining a space or cavity (36) between the fingers, the lumen (28) terminating within the cavity (36), and the cavity (36) being adapted to receive into the cavity an end (8) of a primary fluid conveying system (4) to communicate with the lumen (28) so that fluid may flow through the lumen (28) and into the primary system (4); and

a collar (38) manually slidable along the connector element (22) between a retracted position enabling the fingers (32,34) to lie in a first position in which an end (8) of the primary system (4) may be received into the cavity, and an advanced position flexing the fingers (32,34) to trap and lock the end of the primary system (4) between the fingers (32,34) in the cavity (36).

2.  A connector according to Claim 1 wherein a greater force is required to cause outward flexing of the distal ends of the fingers (32,34) than is required to cause inward flexing of the distal ends of the fingers (32,34).

3.  A connector according to Claim 1 or 2 in which each finger (32,34) has a distal portion or segment (58) connected to the base (26) by a hinge region (62,64) for providing flexing of the fingers (32,34) inwardly.

4.  A connector according to Claim 3 in which the hinge region (62,64) is less rigid than the distal portion or segment (58) of the finger (32,34).

5.  A connector according to Claim 3 or 4 in which the collar (38) in the said retracted position thereof has portions (42,44) overlying at least partially the hinge region (62,64), to prevent or inhibit the finger (32,34) from flexing outwardly.

6.  A connector according to any preceding claim in which the collar (38) is deformable outwardly to accommodate sliding movement of the collar (38) when engaging distal portions of the fingers (32,34).

7.  A connector according to any preceding claim including a cam (66) on the exterior surface of each finger (32,34) engageable by the collar (36) in response to sliding movement of the collar towards the said advanced, locking position.

8.  A connector according to any preceding claim in which the base (26) includes a cannula (30) projecting generally axially from the base (26) relative to a main axis of the connector element (12), with the said lumen (28) extending through the cannula (30), the said fingers (32,34) projecting from the base (26) in a generally axial direction and the said cavity (36) opening in the said axial direction through a distal end of the connector element (22), the cannula (30) extending from the base (26) towards the said distal end of the connector element (22) and terminating within the cavity (36),

the collar (38) being slidable between the said retracted position, in which position a junction terminal (8) with a septum (9) at an end thereof may be received in the cavity (36) through the opening thereof and the septum (9) may be penetrated by the cannula (30), and the said advanced position, in which position the fingers (32,34) trap and lock the junction terminal (8) between the fingers in the cavity (36).

9.  A connector according to any preceding claim in which the collar includes first and second bars (42,44) projecting therefrom in a direction towards the distal end of the connector element (22), and there being provided a cam (66) on each of the fingers (32,34) engagable with the bars (42,44) in response to sliding movement of the collar (38) towards the said advanced position to deflect the fingers (32,34) inwardly towards one another, the bars (42,44) being flexible outwardly when the fingers (32,34) are inhibited from flexing inwardly by engagement thereof with an end (8) of a primary fluid conveying system (4).

10. A connector according to any preceding claim in which the fingers are (32,34) spaced one from the other to define slots there between, and the collar (38) has a shield portions (46) overlying at least parts of the said slots in response to movement of the collar (38) into the said advance locking position to inhibit access to the interior of the connector element (22).

11. A medical connector for manually connecting two fluid conveying systems, comprising:

a connector element (22) having a base (26) with a passage or lumen (28) extending through the base (26), the base (26) having one end adapted to be connected to a secondary fluid conveying system (10) in such a manner that fluid received from the secondary system (10) may flow through the lumen (28), the connector element (22) further having at least two fingers (32,34) projecting from the base (26) and defining a space or cavity (36) between the fingers, the lumen (28) terminating within the cavity (36), and the cavity (36) being adapted to receive into the cavity an end (8) of a primary fluid conveying system (4) to communicate with the lumen (28) so that fluid may flow through the lumen (28) and into the primary system (4); and

a collar (38) manually slidable along the connector element (22) between a retracted position enabling the fingers (32,34) to lie in a first position in which an end (8) of the primary system (4) may be received into the cavity, and an advanced position flexing the fingers (32,34) to trap and lock the end of the primary system (4) between the fingers (32,34) in the cavity (36);

in which the fingers (32,34) have distal, relatively rigid, finger portions or segments (58), and the connector element (22) has a hinge region (62,64) proximal to the rigid finger segments (58), the connector element (22) being more easily deformed along the hinge region (62,64) than along the distal finger segments (58).

12. A medical connector for manually connecting two fluid conveying systems, comprising:

a connector element (22) having a base (26) with a passage or lumen (28) extending through the base (26), the base (26) having one end adapted to be connected to a secondary fluid conveying system (10) in such a manner that fluid received from the secondary system (10) may flow through the lumen (28), the connector element (22) further having at least two fingers (32,34) projecting from the base (26) and defining a space or cavity (36) between the fingers, the lumen (28) terminating within the cavity (36), and the cavity (36) being adapted to receive into the cavity an end (8) of a primary fluid conveying system (4) to communicate with the lumen (28) so that fluid may flow through the lumen (28) and into the primary system (4); and

a collar (38) manually slidable along the connector element (22) between a retracted position enabling the fingers (32,34) to lie in a first position in which an end (8) of the primary system (4) may be received into the cavity, and an advanced position flexing the fingers (32,34) to trap and lock the end of the primary system (4) between the fingers (32,34) in the cavity (36);

in which the base (26) includes a cannula (30) projecting generally axially from the base (26) with the said lumen (28) extending through the cannula, the said fingers (32,34) projecting from the base in a generally axial direction and the said cavity (36) opening in the said axial direction through a distal end of the connector element (22), the cannula (30) extending from the base (26) towards the said distal end of the connector element (22) and terminating within the cavity (36),

the collar (38) being slidable between the said retracted position, in which position a junction terminal (8) with a septum (9) at an end thereof may be received in the cavity (36) through the opening thereof and the septum (9) may be penetrated by the cannula, and the said advanced position, in which position the fingers (32,34) trap and lock the junction terminal (8) between the fingers in the cavity,

each of the fingers (32,34) having a distal segment (58) and a connecting segment (62,64) intermediate the base (26) and the distal segment (58), the connecting segment (62,64) defining a hinge region, the said collar (38) engaging the fingers in response to sliding movement of the collar (38) on the element (22) to flex the fingers (32,34) inwardly along the said hinge region (62,64) during movement of the collar (38) to the advanced position.

13. A medical connector for manually connecting two fluid conveying systems, comprising:

a connector element (22) having a base (26) with a passage or lumen (28) extending through the base (26), the base (26) having one end adapted to be connected to a secondary fluid conveying system (10) in such a manner that fluid received from the secondary system (10) may flow through the lumen (28), the connector element (22) further having at least two fingers (32,34) projecting from the base (26) and defining a space or cavity (36) between the fingers, the lumen (28) terminating within the cavity (36), and the cavity (36) being adapted to receive into the cavity an end (8) of a primary fluid conveying system (4) to communicate with the lumen (28) so that fluid may flow through the lumen (28) and into the primary system (4); and

a collar (38) manually slidable along the connector element (22) between a retracted position enabling the fingers (32,34) to lie in a first position in which an end (8) of the primary system (4) may be received into the cavity, and an advanced position flexing the fingers (32,34) to trap and lock the end of the primary system (4) between the fingers (32,34) in the cavity (36);

in which the base (26) includes a cannula (30) projecting generally axially from the base (26) with the said lumen (28) extending through the cannula, the said fingers (32,34) projecting from the base in a generally axial direction and the said cavity (36) opening in the said axial direction through a distal end of the connector element (22), the cannula (30) extending from the base (26) towards the said distal end of the connector element (22) and terminating within the cavity (36),

the collar (38) being slidable between the said retracted position, in which position a junction terminal (8) with a septum (9) at an end thereof may be received in the cavity (36) through the opening thereof and the septum (9) may be penetrated by the cannula, and the said advanced position, in which position the fingers (32,34) trap and lock the junction terminal (8) between the fingers in the cavity,

in which each of said fingers has a distal segment (58) and a connecting segment (62,64) intermediate said base (26) and said distal segment (58) and defining a hinge region, said hinge region (62,64) of the connecting segment of each finger (32,34) being less rigid than the distal segment thereof, the collar (38) including first and second bars (42,44) projecting therefrom in a direction towards the distal end of the connector element (22), and there being provided a cam (66) on each of the fingers engagable with the bars (42,44) in response to sliding movement of the collar (38) towards the said advanced position to deflect the fingers (32,34) inwardly towards one another, the bars (42,44) being flexible outwardly when the fingers are inhibited from flexing inwardly by engagement thereof with an end (8) of a primary fluid conveying system (4).

14. A medical connector for manually connecting two fluid conveying systems, comprising:

a connector element (22) having a base (26) with a passage or lumen (28) extending through the base (26), the base (26) having one end adapted to be connected to a secondary fluid conveying system (10) in such a manner that fluid received from the secondary system (10) may flow through the lumen (28), the connector element (22) further having at least two fingers (32,34) projecting from the base (26) and defining a space or cavity (36) between the fingers, the lumen (28) terminating within the cavity (36), and the cavity (36) being adapted to receive into the cavity an end (8) of a primary fluid conveying system (4) to communicate with the lumen (28) so that fluid may flow through the lumen (28) and into the primary system (4); and

a collar (38) manually slidable along the connector element (22) between a retracted position enabling the fingers (32,34) to lie in a first position in which an end (8) of the primary system (4) may be received into the cavity, and an advanced position flexing the fingers (32,34) to trap and lock the end of the primary system (4) between the fingers (32,34) in the cavity (36);

in which the base (26) includes a cannula (30) projecting generally axially from the base (26) with the said lumen (28) extending through the cannula, the said fingers (32,34) projecting from the base in a generally axial direction and the said cavity (36) opening in the said axial direction through a distal end of the connector element (22), the cannula (30) extending from the base (26) towards the said distal end of the connector element (22) and terminating within the cavity (36),

the collar (38) being slidable between the said retracted position, in which position a junction terminal (8) with a septum (9) at an end thereof may be received in the cavity (36) through the opening thereof and the septum (9) may be penetrated by the cannula, and the said advanced position, in which position the fingers (32,34) trap and lock the junction terminal (8) between the fingers in the cavity,

in which the fingers (32,34) Oare spaced one from the other to define slots there between, and the collar (38) has a shield portions (46) overlying at least parts of the said slots in response to movement of the collar (38) into the said advance locking position to inhibit access to the interior of the connector element (32) to the slots to inhibit contamination.

# FIG. 1a

# FIG. 1b

# FIG. 1c

# FIG. 1d

# FIG. 1e

# FIG. 1f

FIG. 2A

FIG. 2B

**FIG. 3**

**FIG. 4**

**FIG. 5**

**FIG. 6**

FIG. 7

FIG. 8

FIG. 9

FIG. 10

**FIG. 11**

**FIG. 12**

**FIG. 13**

**FIG. 14**

FIG. 15

FIG. 16

## FIG. 17a

## FIG. 17b

EP 0 453 264 A1

| | European Patent Office | EUROPEAN SEARCH REPORT | Application Number |
|---|---|---|---|

EP 91 30 3429

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | US-A-4 895 570 (LARKIN)<br>* column 2, line 57 - line 68 *<br>* column 5, line 16 - line 22; figures 1-4 * | 1,8 | A61M39/00<br>F16L37/12 |
| Y | | 2-7,9-14 | |
| Y | EP-A-0 031 022 (INTERMEDICAT GMBH)<br><br>* figures 1,2 * | 3-7,9,<br>11-13 | |
| Y | FR-A-2 491 192 (FESTO-MASCHINENFABRIK GOTTLIEB STOLL)<br>* page 7, line 16 - line 18; figure 1 * | 2 | |
| Y | US-A-4 573 713 (KIPP ET AL.)<br>* figure 1B * | 10,14 | |
| A | US-A-4 398 757 (FLOYD ET AL.)<br>* figure 4 * | 7,13 | |
| | | | **TECHNICAL FIELDS SEARCHED (Int. Cl.5 )**<br><br>A61M<br>F16L |

The present search report has been drawn up for all claims

| Place of search | Date of completion of the search | Examiner |
|---|---|---|
| THE HAGUE | 13 JUNE 1991 | SEDY, R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

22